# EUROPEAN PATENT APPLICATION

(11) **EP 3 354 224 A1**
(43) Date of publication of application: **01.08.2018**
(21) Application number: 16794498.2
(22) Date of filing: 22.09.2016
(51) Int. Cl.: A61B 90/10, A43D 1/02, A61B 5/00, A61B 5/107

(54) **STEREOTACTIC FRAME FOR LIMBS**

(30) Priority: 24.09.2015 CU 20150132
(71) Applicant: Centro de Ingeniería Genética y Biotecnología, La Habana 11600 (CU)
(72) Inventor: CABAL MIRABAL, Carlos, Alberto, La Habana 10400 (CU); GONZÁLEZ DALMAU, Evelio, Rafael, La Habana 10800 (CU); ORAMAS DIAZ, Leonardo, La Habana 11500 (CU); HERRERA MARTINEZ, Luis, Saturnino, La Habana 11600 (CU); BERLANGA ACOSTA, Jorge, Amador, La Habana 11600 (CU); FERNÁNDEZ MONTEQUÍN, José, Ignacio, La Habana 10 000 (CU); GUILLÉN NIETO, Gerardo, Enrique, La Habana 10400 (CU); FLORES DÍAZ, Luis, Manuel, La Habana 11 600 (CU); CABAÑAS RODRÍGUEZ, Orestes, Lucio, La Habana 11 600 (CU); GONZALEZ BLANCO, Sonia, La Habana 11 600 (CU); UBIETA GÓMEZ, Raimundo, La Habana 11 600 (CU)
(74) Representative: V.O.
(86) International application number: PCT/CU2016/050002
(87) International publication number: WO 2017/050302

(57) **Abstract**

The present invention is related to a stereotaxic frame for extremities that ensures that the extremity position is fixed and reproducible; keeping the same geometric conditions of the measurement or treatment devices regards the extremity, or part thereof, during the study or therapy. The frame comprises a positioning and fixing holder for the extremity and a measuring or treatment system, that includes a geometric locus where the measurement or treatment device is allocated, ensuring that the relative position of said device regards the region of the extremity under examination or treatment is reproducible.

## Description

### Technical field

The present invention is related to the fields of human and veterinary medicine, the pharmaceutical industry and biomedical research. It is applicable to sport and work medicine, endocrinology, orthopedics, rheumatology, angiology, dermatology and traumatology, among others medical specialties. It allows performing quantitative evolutional photographic, optical, thermographic studies without contacting the extremity or limb, by fixation and reproduction of its relative position with regards to the measurement device or to those that are employed for the treatment of extremity conditions. In addition, it enables successive treatment of the limbs, where their position is essential, without contacting them.

### Previous art

A large set of inflammatory, degenerative, traumatic, infectious, autoimmune, orthopedic, vascular or neurological diseases affect the anatomy and physiology of the human extremities, like hands, feet and lower parts of the legs. The causes of these conditions and their treatment are quite diverse. The extremity diseases disturb, in varying degrees, the rest of the body. A growing number of people subject the extremities to high stress, due to professional activities or accidents. Among said people are builders, miners, farmers, sportsmen and sportswomen, artists and militaries.

On other hand, there are multiple (and high incidence) conditions affecting the higher and lower extremities, such as diabetic foot ulcers (DFU), rheumatoid arthritis, deformities, inflammations and infections, circulatory, traumatic, and neuropathic conditions, among others. The methods of diagnosis, monitoring and treatment of said conditions of the extremities are still insufficient.

Some papers and patent documents have addressed the study of the lower extremities *(Patent* US6834437*; Patent* EP1902640*; Patent application* US20110118630*; Patent application* US20130053677*; Patent application* US20060225297*; Patent application* US20130258085*; Patent application* US20130114869*; Patent* CA1309192*; Patent application* US20090003531*).* Several of these documents are limited to expose systems to measure the dimensions of the feet, for purposes of design of specialized footwear. In other cases *(Patent application* US20060225297*, Patent* EP1902640*),* measurement systems are limited to the foot soles, by contacting them to assess tread defects. In addition, there is a disclosed scanning and measuring system of the foot soles (*Patent Application* US20130053677)*,* for evaluation of plantar DFU in two and three dimensions. This system has severe disadvantages, such as: a) the measuring system involves contact with the feet, even when said limb have ulcers, that are usually infected, painful and deformed when contacted with the device; and b) the device is only useful to evaluate ulcers localized in the plantar region.

Recently, a paper (Wang L et al., J. Diabetes Sci Technol 1-8, 2015) disclosed a mobile phone, placed in a box, to photograph and transmit images of the DFU, to be digitally processed and evaluated. This publication takes in accounts only plantar ulcers, excluding the lesions in any other part of the foot and leg. In addition, the device comes into contact with the lesion, deforming it, does not guarantee the reproducibility of the feet positioning, and it restricts the research to such type of pathologies.

Another measuring device (*Patent application* US20110118630) is restricted to evaluation of the mechanical torque of the foot, around the axis of the leg for orthopedic studies. The apparatus to fasten the feet in another system (*Patent application* US20090003531) refers to a foot fastening device employed during simple X-ray examination.

The mentioned works have essential limitations in their benefits: a) make contact with the examined foot (which makes them invasive, of non-reliable hygiene and uncomfortable for the patient), and produce deformation of the studied part by the mechanical contact; b) the measurements are restricted to the foot soles; c) do not guarantee the reproducibility of positioning; d) do not allow evolutional and transversal quantitative studies; and e) employ a single type of sensor devices. Quantification of the variables that characterize the anatomical and physiological processes in the extremities and their parts, to provide qualitative- and quantitatively new information and evolutional information, it is not solved with any of these inventions.

The problem of evolutional and quantitative transversal assessments studies, of various pathologies of the lower and upper limbs requires achieving a fixed and reproducible position throughout different tests. It includes inflammatory processes (that alter the size and relative location of the anatomical structures). Having quantitative information (two-dimensional and three-dimensional) of existing pathophysiological processes in all parts of the extremities, and its evolution, through multiple sensors, is an unsolved problem, and its solution opens new possibilities and paradigms in the study and treatment of the extremities

### Description of the invention

The present invention solves the problem mentioned above, by providing a stereotaxic frame for the extremities, comprising a positioning and fixing holder of the extremity (5) and a measurement or treatment system, comprising at least a measurement or treatment device (1) located at a geometrical locus (2), that guarantee that the relative position of said device respect to the region of the extremity under exam or treatment is reproducible.

With the stereotaxic frame of the invention, the same geometric conditions of the measurement or treatment devices respect to the extremity, or its part, during several studies, or therapy sessions, for the same patient or individual, are preserved. It also permits the comparative quantitative evaluation, between different individuals or patients, since it secures similar and reproducible conditions of study. It allows measurements (of area, volume, texture, color, temperature, vascularization, etc.) in the surface of the extremity, or in its anatomic regions. In addition, it permits the treatment of lesions present in said extremities.

The quantitative bidimensional and tridimensional information is essential to evaluate the evolution of the limbs with and without conditions, and for the characterization of pathologies under study, as well as to assess the effectiveness of the treatment schedules of diseases that affect the patient lower and upper limbs.

The invention described herein permits the simultaneous multisensory study of any part of the extremity, without contacting the anatomical parts or lesions under investigation, ensuring non-invasive, hygienic and comfortable studies, both for the patients and for the operators. The possibility of making a multisensory study allows quantitative bidimensional and tridimensional measurements, of multiple parameters, with very low dispersion, within the same study. The measurements conducted within the invention offer a more complete, integral, and exact view of the evolution of parts and lesions examined as time passes, either if the person is on treatment or not. It is applicable for healthy individuals or for patients under study.

In an embodiment of the invention, in the stereotaxic frame is ensured the reproducibility of the relative position of the measurement or treatment device respect to the region under exam, only fixing two variables.

In an embodiment of the invention, the positioning and fixing holder of the extremity and the measurement or treatment system are located on a base (10). In a preferred embodiment, the measurement or treatment system comprises: i) the geometrical locus (2), where the measurement or treatment device is located (1), ii) adjustment elements (8) that allow that the geometrical locus rotate around an axis (7), iii) reference system (6), iv) the system for signaling and recording the relative position of the measurement or treatment device on the geometrical locus and v) at least one measurement or treatment (1).

To ensure the reproducible position of the extremity, in the longitudinal and transversal studies, the frame is provided with a reference system (6), that guides the act of positioning of the extremity and locating of the zone of interest for the study, in a fix and reproducible position of the limb in the stereotaxic frame.

In an embodiment of the invention, the positioning and fixing holder comprises an elevation or vertical movement device (11) for the extremity under exam or therapy. In a particular embodiment, the positioning and fixing holder of the extremity is made of a resistant, non-rough, relatively light material, to ensure its preservation, facilitate its transfer, and allow its cleaning and disinfection. The holder for positioning the extremities can be covered by a soft material that provides comfort to the individual under the study. The holder can comprise means to fasten the extremity in it. Likewise, if an upper extremity is under study, the proper complement is coupled to the positioning and fixing holder (5).

On the other hand, in an embodiment of the invention, the geometrical locus (2) where the measurement or treatment device (1) is located is an arc. In a particular embodiment, the angle of rotation of the arc around the axis (7) is recorded in a scale (9).

In an embodiment of the invention, the measurement device present in the stereotaxic frame comprises at least one sensor. The invention solves a problem present in this technical field, as it provides an stereotaxic frame for positioning, fixing and register, to control the location of the extremities of an individual under observation, respect to the system of sensors used for the bidimensional and tridimensional evolutional quantitative study of the limbs, their anatomical parts or lesions, ensuring that the same relative positions of extremity-measurement system are preserved, along serial (longitudinal) or transversal studies of the extremities. The sensors that are part of the stereotaxic frame of the invention can be photograph cameras, temperature sensors, etc. The information obtained therein is preferably digital, allowing its transmission, post processing, storage, the creation of data bases and the remote transmission.

In a particular embodiment, the sensors are located in the geometrical locus (2) of the points equidistant of the extremity (3 and 4), and they glide through said geometrical locus to allow the observation and register of the different parts of the extremity of interest for the study. The location of the sensor in the geometrical locus (2) allows its fixing, and permits the precise register of its position. The geometrical locus (2) rotates around the axis (7), which guarantees its visualization in any part of the extremity. The path around the axis (7) is determined by a visible angle in a scale. The relative position of the measurement system respect to the zone of the extremity to study is determined only by two parameters: The angle in the geometrical locus (2) and the angle around the axis (7). It makes easier the register of the only two position variables, and diminishes the possible human errors.

The stereotaxic frame of the invention allows the study and treatment of the upper and lower extremities. It also admits the treatment of the patient while its extremity is fixed through the stereotaxic frame. In an embodiment of the invention, the position of the extremity is fixed, in a reproducible manner, to allow the treatment of lesions or conditions present in it, through different interventions. The properties of the frame of the invention make possible its use in medical procedures where is required that the patient is in a fix and reproducible position. For example, the frame of the invention can be employed in the thermotherapy, laser microsurgery, ultrasonic wave radiation, etc.

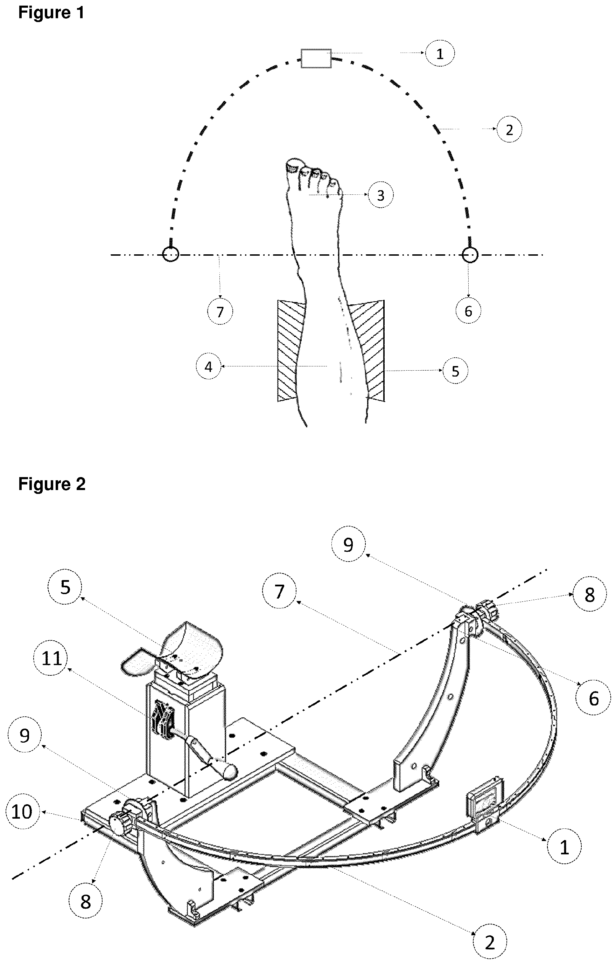

To achieve better comprehension of the invention, in Figure 1 is represented the general scheme of the stereotaxic frame thereof. Figure 2 is the isometric view of one of the embodiments of said frame, with its essential parts, but it does not constitute a limitation to the invention.

Another object of the invention is a method for the study of the upper and lower extremities comprising: positioning the extremity, employing the stereotaxic frame in the quantitative study, and processing the acquired information. In an embodiment of the invention, the acquired information is in digital format; allowing its transmission, post-processing, storage, creation of data bases and remote transmission.

Another aspect of the invention is a method for the treatment of conditions of the extremities, comprising the use of the stereotaxic frame described before.

### Brief description of the figures

**Figure 1****.** General scheme of the stereotaxic frame.
**Figure 2****.** Isometric view of one embodiment of the invention.

### Detailed description of the invention. Examples.

To achieve a better understanding of the invention, without being limited thereto, in Figure 1 the main elements of the stereotaxic frame for extremities are indicated: **1.** Measuring or treatment device, **2.** Geometric locus that ensures the reproducibility of the relative position of the measuring or treatment device in relation to the region under examination or treatment, only fixing two variables, **3.** and **4.** Extremity under study and/or treatment. **5.** Positioning and fixing system for the extremity under study and / or treatment, **6.** Reference system for localization of the extremity under study and/or treatment in the correct position. **7.** Symmetry axis around which rotates the locus of all the points equidistant from the region to study and/or treat. Similarly, if the upper extremity is studied it is only necessary to couple the proper holder to the positioning and fixing system.

To use the frame of the invention, for example, the individual lying face up (supine position) on the examination table put the leg in the system of positioning and fixing (5), which is suitably fixed to prevent inadvertent movements of the member. The device (or devices) for measuring or treatment (1) is positioned on the geometric locus (2), and it can be moved in it, if needed. The displacement of the measuring or treatment device around the geometric locus (2) is recorded on a scale incorporated therein. Furthermore, the geometric locus (2) can rotate around the axis (7), as appropriate, to display the portion of the limb to evaluate or treat. Similarly, if the upper extremity is studied, the proper receptacle is coupled to the positioning and fixing holder (5).

In Figure 2, in an isometric view, the main parts of a stereotaxic frame for extremity studies are indicated. Said frame is an embodiment of the present invention. The person lying face up (supine position) on the examining table places his extremity in the positioning and fixing system (5), which is suitably fixed to prevent unintentional movement. The stereotaxic frame of the invention has a vertical movement or elevation device (11), to ensure adequate, comfortable and reproducible position of the limb under examination and / or treatment. In Figure 2, a reference system suitably positioned for locating the position of the extremity under study or treatment is represented by (6). In the case of this embodiment, the reference system are led diodes as luminous markers. The sensor or sensors represented by (1) is placed on the geometric locus (2), which in this embodiment of the invention is an arc that ensures that the sensors are equidistant from the region under examination. The sensor or sensors can be moved through the arc (2), as convenient. The displacement of sensor or sensors around the arc (2) is recorded on a scale incorporated in said arc. With the adjustment elements denoted like (8) the position of the arc is released or fixed (2), in its rotation around the axis (7). The arc (2) can rotate around the axis (7), as appropriate, to situate the part of the limb to be evaluated. The arc rotation angle (2) around the axis (7) is recorded on a scale, present in the element denoted as (9). All parts and elements described above are fixed to the base (10). Similarly, if the upper extremity is under study the proper attachment is coupled to the holder for positioning and fixing (5). Once the limb under study, the position of the arc (2) with respect to the axis (7), and the place on the arc (2) where the sensor or sensors will be located, are in a proper position, the measurement object of the limb study is conducted.

Similarly, to use the frame of the invention in the treatment of a limb condition, once that: the extremity position, the wanted arc (2) position respect to the axis (7), and the place on the arc (2) where the element for therapy will be situated has been secure the therapy required for the treatment of the extremity is applied.

## Claims

1. Stereotaxic frame for extremities comprising a positioning and fixing holder of the extremity (5) and a measurement or treatment system, comprising at least a measurement or treatment device (1), located at a geometrical locus (2) that guarantee that the relative position of said device respect to the region of the extremity under exam or treatment is reproducible.

2. The stereotaxic frame of claim 1 wherein the reproducibility of the relative position of the measurement or treatment device, respect to the region under exam or treatment, is achieved by fixing only two variables as maximum.

3. The stereotaxic frame of claim 1 wherein the positioning and fixing holder of the extremity and the measurement or treatment system are located on a base (10).

4. The stereotaxic frame of claims 1 to 3 wherein the measurement or treatment system comprises: i) the geometrical locus (2), wherein the measurement or treatment device (1) is located, ii) adjustment elements (8) that allow that the geometrical locus rotate around an axis (7), iii) reference system (6), iv) the system for signaling and register of the relative position of the measurement or treatment device on the geometrical locus, and v) at least one measurement or treatment device (1).

5. The stereotaxic frame of claim 4 wherein the positioning and fixing holder of the extremity comprises a vertical movement or elevation device (11) of the extremity under exam.

6. The stereotaxic frame of claim 5 wherein the geometrical locus where the measurement or treatment device is located is an arc.

7. The stereotaxic frame of claim 6 wherein the angle of rotation of the arc around the axis (7) is recorded in a scale (9).

8. The stereotaxic frame of claim 1 wherein the extremity under exam or treatment is an upper or lower extremity.

9. The stereotaxic frame of claim 4 wherein the information registered with the measurement device is digital.

10. The stereotaxic frame of claim 4 wherein the measurement device comprises at least one sensor.

11. Method for the study of extremities comprising the use of the stereotaxic frame of any of the claims 1-10.

12. Method for the treatment of extremities comprising the use of the stereotaxic frame of any of the claims 1-10.
